# EUROPEAN PATENT APPLICATION

(11) **EP 4 235 679 A1**
(43) Date of publication of application: **30.08.2023**
(21) Application number: 20940476.3
(22) Date of filing: 06.11.2020
(51) Int. Cl.: G16B 50/00, G06F 9/54

(54) **SHARED MEMORY-BASED GENE ANALYSIS METHOD AND APPARATUS, AND COMPUTER DEVICE**

(30) Priority: 22.10.2020 CN 202011139824
(71) Applicant: BGI Genomics Co., Ltd., Yantian District Shenzhen, Guangdong Province 518083 (CN); BGI Health (HK) Company Limited, Hong Kong 999077 (HK)
(72) Inventor: YANG, Jiaobo, Shenzhen, Guangdong 518000 (CN); SONG, Chao, Shenzhen, Guangdong 518000 (CN); YU, Chuang, Shenzhen, Guangdong 518000 (CN); ZHANG, Youjin, Shenzhen, Guangdong 518000 (CN); HE, Zengquan, Shenzhen, Guangdong 518000 (CN); WANG, Jin´an, Shenzhen, Guangdong 518000 (CN)
(74) Representative: Hoffmann Eitle
(86) International application number: PCT/CN2020/127072
(87) International publication number: WO 2022/082878

(57) **Abstract**

A shared memory based gene analysis method, apparatus and computer device. The method comprises: reading sample data and preprocessing the sample data; performing a gene analysis on the sample data preprocessed, and determining whether a required library file in the gene analysis is in a gene shared memory; if yes, obtaining the required library file from the gene shared memory, mapping the required library file to a process of the gene analysis of the sample data preprocessed, and completing a corresponding analysis. In this method, a shared memory mechanism is adopted to establish indexes for the gene analysis. Whether a library file that are frequently used in the gene analysis process are in the gene shared memory is determined; if yes, the library file can be obtained from the gene shared memory and can be mapped to the sample data, and can be conveniently mapped from the gene shared memory to an analysis process performed on the sample data. The method can greatly reduce the time and I/O occupation for loading the library file from a hard disk. Therefore, the efficiency of analysis can be improved.

## Description

### CROSS-REFERENCE TO RELATED APPLICATIONS

This application claims the priority of Chinese Patent Application No. 202011139824.9, filed to the Patent Office of the People's Republic of China on Oct. 22, 2020, entitled " SHARED MEMORY BASED GENE ANALYSIS METHOD, APPARATUS AND COMPUTER DEVICE", the entire content of which is incorporated herein by reference.

### TECHNICAL FIELD

The present disclosure relates to the technical field of data processing, in particular to a shared memory based gene analysis method, apparatus, computer device, and a computer-readable storage medium.

### BACKGROUND

With the smooth implementation of the Human Genome Project and the rapid development of sequencing technology, the cost of sequencing has been significantly reduced, and the speed of sequencing has been significantly improved. The cost of the sequencing of human whole genome has been reduced to less than $1000, and the amount of DNA sequence data has increased exponentially. How to utilize and express the of data quickly, then analyze and explain potential problems in gene sequences, and discover information beneficial to human beings from massive data has become an urgent problem to be solved. The more and more applications of sequence data generated by human whole genome sequencing (WGS) and the continuous demand for rapid analysis and processing of massive sequence data have formed a new technical bottleneck for data analysis, which restricts the clinical application of second-generation sequencing technology.

At present, there are many kinds methods and tools for data analysis of the second-generation sequencing in the field of bioinformatics internationally. The most commonly used process mainly comprises an input of data, a preprocessing operation, a sequence comparison, a annotation, a variant calling and a pathway analysis. However, it is very time-consuming to apply the whole process in WGS. In addition, samples input need customized processes such as merging the samples, splitting the samples and so on which need to be performed separately, so that the operation efficiency is low and the I/O consumption is increased. In addition, in the process of data analysis, index files should be loaded separately for each step of analysis and processing. If multiple tasks load the same index file, the tasks will consume more memory and take more time.

### SUMMARY

In view of this, the disclosure provides a shared memory based gene analysis method, apparatus, computer device, and a computer-readable storage medium to solve a technical problem of the low operation efficiency caused by the requirement of the processes such as merging the input samples in some pipelines, the high memory consumption and the high time consumption caused by loading of index files repeatedly in the data analysis process in the prior art.

Some embodiments of this disclosure provide a shared memory based gene analysis method, comprising: reading sample data and preprocessing the sample data; performing a gene analysis on the sample data preprocessed, and determining whether a required library file in the gene analysis is in a gene shared memory; if yes, obtaining the required library file from the gene shared memory, mapping the required library file to a process of the gene analysis of the sample data preprocessed, and completing a corresponding analysis.

Optionally, the method further comprises: determining whether the required library file meets a load condition, in a case where the required library file in the gene analysis is not in the gene shared memory; and loading the required library file into the gene shared memory, in a case where the loading condition is met.

Optionally, determining whether the required library file meets a load condition, in a case where the required library file in the gene analysis is not in the gene shared memory, and loading the required library file into the gene shared memory, in a case where the loading condition is met comprises: acquiring information of the required library file and information of the gene shared memory, wherein the information of the required library file comprises a space required by the required library file and the number of historical load requests, and the information of the gene shared memory comprises a remaining space of the gene shared memory; and if the number of historical load requests is greater than a first preset number, and the space required by the required library file is less than the remaining space of the gene shared memory, loading the required library file into the gene shared memory.

Optionally, the information of the required library file further comprises a load request frequency of the required library file, the information of the gene shared memory further comprises load request frequencies of all library files; determining whether the required library file meets a load condition, and loading the required library file into the gene shared memory, in a case where the loading condition is met further comprises: if the number of historical load requests is greater than the first preset number, and the space required by the required library file is greater than the remaining space of the gene shared memory, ranking the required library file and the all library files in an order of priority according to the load request frequency of the required library file and the load request frequencies of the all library files to obtain a load request frequency priority of each library file; if the load request frequency priority of the required library file is higher than that of a library file in the gene shared memory, and if the remaining space of the gene shared memory after deleting the library file with a lower load request frequency priority in the gene shared memory is greater than or equal to the space required by the required library file, deleting the library file with the lower load request frequency priority in the gene shared memory; and loading the required library file into the gene shared memory.

Optionally, the method further comprises: setting the gene shared memory for library files used in gene analysis, setting a size of the gene shared memory, the number of library files that can be accommodated, a name of each library file and a size offset of the each library file; and loading library files commonly used in gene analysis into the gene shared memory according to the size of the gene shared memory, the number of library files that can be accommodated, the name of the each library file and the size offset of the each library file.

Optionally, the gene analysis comprises an alignment analysis, a variation analysis and an annotation analysis, the method further comprises: performing the alignment analysis, the variation analysis, and the annotation analysis on the sample data preprocessed in sequence, wherein in a case where the sample data preprocessed comprises multiple groups of sample data, the multiple groups of sample data are in a same step or different steps of the gene analysis at a time.

Optionally, the gene analysis further comprises a sorting analysis and a marking-duplicate analysis, wherein after performing the alignment analysis, the variation analysis, and the annotation analysis on the sample data preprocessed in sequence, the method further comprises: labeling the sample data after the alignment analysis with a position tag; and performing the sorting analysis and the marking-duplicate analysis by module on the sample data labeled.

Optionally, the method further comprises: connecting some or all steps of the gene analysis by a use of memory.

Optionally, preprocessing the sample data comprises: performing a quality control, a filtering operation and a statistical process on the sample data.

Some embodiments of the disclosure also provide a gene shared memory based gene analysis apparatus, comprising: a data reading module configured to read sample data; a data preprocessing module configured to preprocess the sample data; and a gene analysis module configured to perform a gene analysis on the sample data preprocessed, and determine whether a required library file in the gene analysis is in a gene shared memory; if yes, obtain the required library file from the gene shared memory, map the required library file to a process of the gene analysis of the sample data preprocessed, and complete a corresponding analysis.

Some embodiments of the disclosure further provide a computer device, comprising a memory, a processor and a computer program stored on the memory and executable on the processor. The processor executes the following steps: reading sample data and preprocessing the sample data; performing a gene analysis on the sample data preprocessed, and determining whether a required library file in the gene analysis is in a gene shared memory; if yes, obtaining the required library file from the gene shared memory, mapping the required library file to a process of the gene analysis of the sample data preprocessed, and completing a corresponding analysis.

Some embodiments of the disclosure further provide a computer-readable storage medium on which a computer program is stored, wherein the computer program when executed by a processor implements the following steps: reading sample data and preprocessing the sample data; performing a gene analysis on the sample data preprocessed, and determining whether a required library file in the gene analysis is in a gene shared memory; if yes, obtaining the required library file from the gene shared memory, mapping the required library file to a process of the gene analysis of the sample data preprocessed, and completing a corresponding analysis.

The gene shared memory based gene analysis method, apparatus, computer device and computer readable medium are provided in the embodiments of the disclosure. Sample data is read first, and then the sample data is preprocessed, and then a gene analysis is performed on the sample data preprocessed. In the gene analysis, it is necessary to determine whether a required library file is in a gene shared memory of library files in gene analysis; if yes, the required library file is obtained from the gene shared memory, and mapped to the gene analysis corresponding to the sample data to complete the corresponding analysis . In the gene shared memory based gene analysis method the gene shared memory mechanism is used to establish indexes for gene analysis (for example comprises alignment analysis, variant calling analysis, annotation analysis and so on), and then stores files in a database (i.e. library files) required in the gene analysis in the gene shared memory. A library file can be conveniently mapped from the gene shared memory to a process of the gene analysis performed on the sample data. On one hand, the time and the I/O occupation for loading the library file from a hard disk are greatly reduced. On the other hand, the communications among multiple processes in the process of the gene analysis are facilitated and the repeatedly loading of the library file is avoid.

### BRIEF DESCRIPTION OF THE DRAWINGS

In order to more clearly explain the embodiments of the present disclosure or the technical solutions in the prior art, a brief introduction will be given below for the drawings required to be used in the description of the embodiments or the prior art. It is obvious that, the drawings illustrated as follows are merely embodiments of the present disclosure. For a person skilled in the art, he or she may also acquire other drawings according to such drawings on the premise that no inventive effort is involved.
Fig. 1 is a schematic diagram of an application environment of a shared memory based gene analysis method according to some embodiments of the present disclosure;
Fig. 2 is a flow diagram of a shared memory based gene analysis method according to some embodiments of the present disclosure;
Fig. 3 is a schematic diagram showing a principle diagram of a shared memory according to some embodiments of the present disclosure;
Fig. 4 is a flow diagram of constructing a shared memory in some embodiments of the present disclosure;
Fig. 5 is a structure diagram of a shared memory in some embodiments of the present disclosure;
Fig. 6 is a flow diagram of a shared memory based gene analysis method according to some embodiments of the present disclosure;
Fig. 7 is a diagram showing a CPU utilization and an I/O utilization when a gene analysis is performed using a method A according to some embodiments of the present disclosure;
Fig. 8 is a diagram showing a CPU utilization and an I/O utilization when a gene analysis is performed using a method B according to some embodiments of the present disclosure;
Fig. 9 is a diagram showing a CPU utilization and an I/O utilization when a gene analysis is performed using a method C according to some embodiments of the present disclosure;
Fig. 10 is a structure diagram of a shared memory based gene analysis apparatus according to some embodiments of the present disclosure;
Fig. 11 is a structure diagram of a computer device according to some embodiments of the present disclosure.

### DETAILED DESCRIPTION

The technical solutions in the embodiments of the present disclosure will be clearly and completely described below. Obviously, the described embodiments are only a part of the embodiments of the present disclosure, but not all of the embodiments. All other embodiments obtained by those of ordinary skill in the art based on the embodiments of the present disclosure without creative efforts shall fall within the protection scope of the present disclosure.

### Glossary:

Gene (Mendelian factor) refers to a DNA or a RNA sequence that carries genetic information (that is, a gene is a DNA or a RNA fragment with genetic effects), also known as genetic factor, which is a basic genetic unit that controls biological traits . A gene expresses genetic information it carries by directing a synthesis of proteins, thereby controlling the traits of individual organisms. Gene sequencing is a new type of gene detection technology that analyzes and determines the whole sequence of genes from blood or saliva, so as to predict the possibility of suffering from a variety of diseases, individual behavior characteristics and reasonable behaviors.

Read: A short sequencing fragment, which is sequencing data generated by a high-throughput sequencer. Tens of millions of reads will be generated by sequencing an entire genome. Then, by splicing these reads together, the full sequence of the genome can be obtained.

Alignment analysis : Reads sequenced by NGS are stored in FASTQ files. Although they originally came from an ordered genome, the sequential relationship between different reads in the files has been lost after DNA library building and sequencing. Therefore, there is no positional relationship between two reads next to each other in the FASTQ files. They are all short sequences randomly derived from certain positions in the original genome. Therefore, we need to straighten out a lot of short sequences first, compare them with a reference genome of the species one by one, find the position of each read on the reference genome, and then arrange them in order. This process is called the comparison of sequencing data.

Sorting analysis: Why are BAM files output out of order after a BWA comparison? The reason is that these sequenced reads in the FASTQ files are randomly distributed on the genome. The first step of the comparison is to locate the reads one by one on the reference genome according to their order in the FASTQ files, and then output them directly. It is impossible in this step to automatically recognize the sequence of their comparison positions and rearrange the comparison results. Therefore, in the result file obtained after the comparison, the positional order of the records is chaotic. We need to sort the records in order for a subsequent step such as marking-duplicate, which is the reason for the need to sort.

Marking-duplicate: After the sorting is completed, deduplication is performed (i.e., removing PCR duplicated sequences) . What is a duplicated sequence? How is it produced and why does it need to be removed? It is related to the library construction and sequencing in the experimental process. Before NGS sequencing, a sequencing library needs to be constructed: cut the original DNA sequence by physical (ultrasonic) interruption or using a chemical reagent (enzyme digestion), and then select sequences in a specific length range for PCR amplification and computer sequencing. Therefore, the duplicated sequence here is actually introduced during the PCR process.

Base quality score correction: It is to (as far as possible) correct systematic errors in the sequencing process, because a variant calling is a step that relies heavily on the sequencing base quality scores. Because this quality score is an important (even the only) indicator to measure how correct the base we sequenced is. It cannot be measured directly, but an extremely close distribution result can be obtained through statistical techniques. A known variation found in a population is likely to be the same in someone. Therefore, we can compare and analyze the comparison result directly, exclude all known variation sites, and then calculate how many bases are different from those on the reference genome after comparison for each (reported) quality score. These different bases are considered as wrong bases, and their number ratio reflect the real base error rates, which are converted into Phred scores. This information is output into a calibration table file, and is used to re-adjust the base quality scores in the original BAM file. A new BAM file is output using these new quality scores.

Variant calling and analysis: the purpose of variant calling and analysis is to accurately detect a variation set in the genome of each sample (such as human), that is, those DNA sequences that are different for different people.

In order to make the obj ect, technical solution and advantages of the present application more clear and explicit, the present application will be further described in detail in combination with the drawings and the embodiments. It should be understood that the detailed embodiments that will be described herein are only used for explaining the present application, but not used for limiting the present application.

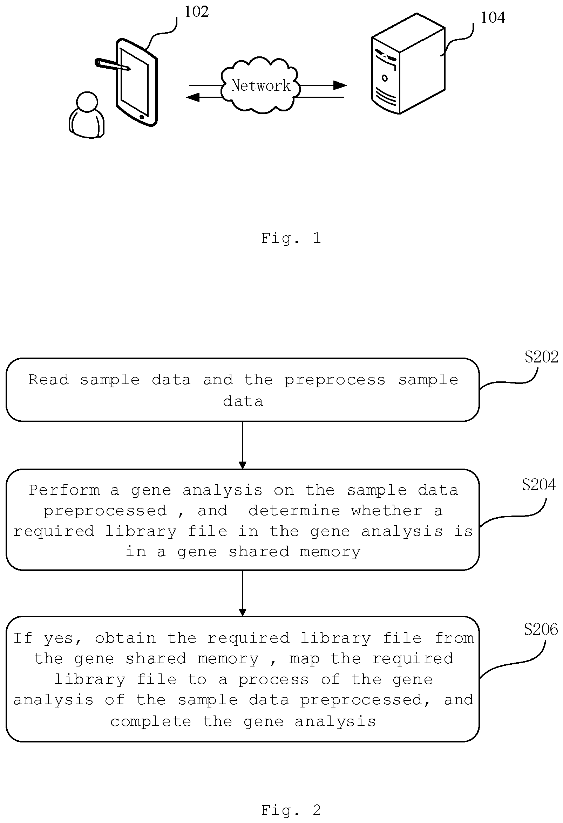

This method can be applied to the terminal 102 in Fig. 1. The terminal can be a personal computer, laptop, etc. The terminal 102 is connected with a gene sequencing device 104, which can be a gene sequencer, etc.

When the terminal 102 is connected with the gene sequencing device 104 through a local interface, the gene sequencing device 104 can send sample data after sequencing to the terminal 102. In addition, the terminal 102 can obtain the sample data after sequencing in the gene sequencing device 104 through instructions.

In some embodiments, as shown in Fig. 2, a shared memory based gene analysis method is provided. As an illustration, this method is applied to the terminal in Fig. 1 as an example, and comprises the following steps:

In step S202, sample data is read and the sample data is preprocessed.

The sample data is data generated or formed after gene sequencing of samples. The number of the samples can be one or more groups.

In an optional embodiment, preprocessing the sample data comprises: performing a quality control, a filtering operation and a statistical process on the sample data.

The data obtained from gene sequencing is called raw data (i.e. raw reads or raw data) . The raw data may contain low-quality sequences and splice sequences, which will affect the analysis result. Therefore, a series of data processing shall be carried out on the raw data, such as a quality control, a filtering operation and a statistical process, to remove impurities in the raw data, so as to determine whether the sequencing data is suitable for subsequent analysis.

In step S204, a gene analysis on the sample data preprocessed is performed, and whether a required library file in the gene analysis is in a gene shared memory is determined.

Generally, after preprocessing the sample data, it is necessary to carry out a relevant gene analysis on the sample data. A common analysis mainly comprises a sequence alignment (i.e. alignment analysis), a variant calling (i.e. variation analysis), a annotation statistics (i.e. annotation analysis) and a subsequent pathway analysis (such as a GO analysis, a KEGG analysis and a protein pathway analysis) . However, no matter which analysis is carried out, it needs to adopt an analysis database. For example, a reference genome database is required for the alignment analysis, a species genome database (such as a human genome database) is required for the variant calling, an annotation database is required for the annotation analysis, a pathway database is required for the pathway analysis, etc. Each database has a large amount of data. These databases need to be loaded when the analysis is carried out on the sample data.

Shared memory is the last way of interprocess communication in System V. Shared memory, as its name implies, allows two unrelated processes to access a same logical memory, and is a very effective way to share and transfer data between two running processes. The memory shared between different processes is usually a same piece of physical memory. Processes can connect the same piece of physical memory to their own address space, and all processes can access addresses in the shared memory. If a process writes data to the shared memory, this change will immediately affect any other process that can access the same piece of shared memory.

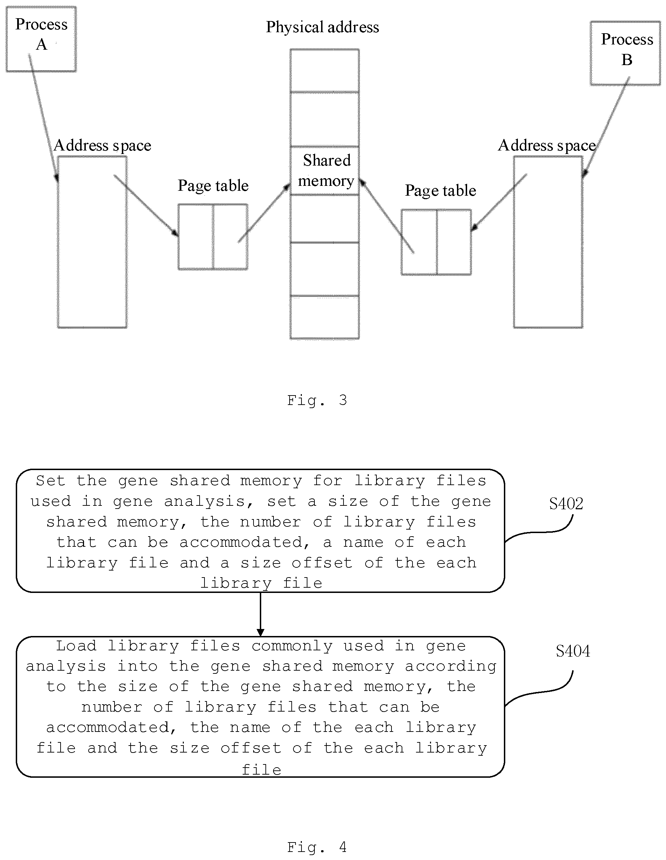

Fig. 3 is a schematic diagram showing the communication principle of a shared memory. In Linux, each process has its own process control block (PCB) and address space (Addr Space), and has a corresponding page table, which is used for mapping virtual addresses of the process to physical addresses and is managed through a memory management unit (MMU). Two different virtual addresses may be mapped to a same area in a physical space by using the page table, and this area they point to is a shared memory. Referring to Fig. 3, there are two processes ProcA and ProcB in the Figure. When virtual addresses are mapped to a physical address through page tables of these two processes, there is a common memory area of the physical address, that is, a shared memory, which can be seen by the two processes at a same time. In this way, when one process writes and another process reads, an inter process communication can be realized between the two processes. For the shared memory, its implementation adopts a principle of reference counting. When a process detaches the shared memory area, a counter decreases by one. When a process successfully hitches to the shared memory area, the counter increases by one. The shared memory area can be deleted only if the counter becomes zero. When the process terminates, the shared memory area attached to it will automatically detach from it.

In the embodiments, a gene shared memory is constructed for library files in gene analysis, in which the most commonly used databases in gene analysis processing can be stored. When a database is needed in an analysis of sample data, it can be obtained directly from the gene shared memory, which greatly reduces time of loading the database from a loading library of a disk. In addition, when multiple groups of sample data are analyzed at a same time, the database can be shared among the multiple groups of sample data, which reduces repeated loading and I/O occupation.

In an optional embodiment, as shown in Fig. 4, there is also provided a method of constructing a shared memory, comprising: steps S402~S404_{∘}

In step S402, the gene shared memory for library files used in gene analysis is set, a size of the gene shared memory, the number of library files that can be accommodated, a name of each library file and a size offset of the each library file are set.

In step S404, library files commonly used in gene analysis are loaded into the gene shared memory according to the size of the gene shared memory, the number of library files that can be accommodated, the name of the each library file and the size offset of the each library file.

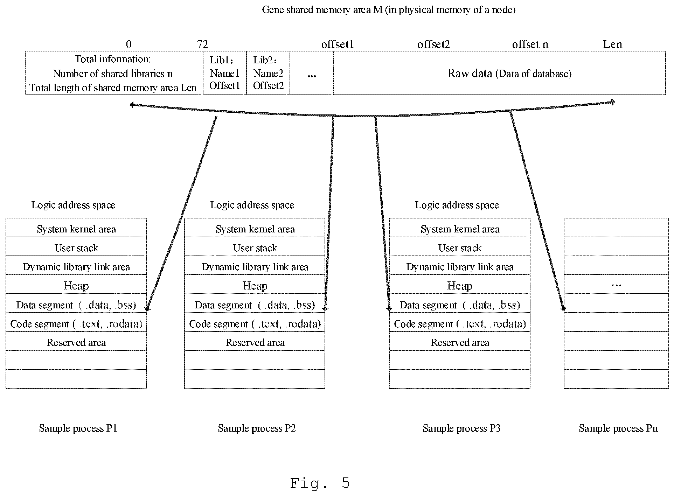

Referring to Fig. 5, a certain area is selected in a terminal system (i.e. a hardware device used for a gene analysis of sample data) as the gene shared memory of library files in the gene analysis. An appropriate size of the gene shared memory is determined according to a storage space, a data processing ability and other performances of the terminal system. Contents recorded or stored in the gene shared memory area mainly comprise: a design of a table header of the gene shared memory in node physical memory: 1) first, store the number (n) of determined shared libraries and a total length (Len) of a shared area; 2) store a name (e.g. Lib1, Lib2) and a length offset (offset1, offset2) of each specified library file in the gene shared memory; 3) store data of the each specified library file in a selected area in turn.

Its working principle is as follows: the sample data can comprise multiple groups of data; each group of data has a corresponding sample process . From sample process P1 to sample process PN, each process has its own process control block (PCB) and address space (Addr space), and has a corresponding page table, which is used for mapping virtual addresses of the process to physical addresses and is managed through a memory management unit (MMU) . Two different virtual addresses may be mapped to a same area in a physical space by using the page table, and this area they point to is a shared memory. Through the above method, each sample process can enter the shared memory area, so as to obtain a required library file in the shared memory area.

In step S206, if yes, the required library file from the gene shared memory is obtained, the required library file is mapped to a process of the gene analysis of the sample data preprocessed, and a corresponding analysis is completed.

In the gene shared memory based gene analysis method provided in the embodiments of the disclosure, Sample data is read first, and then the sample data is preprocessed, and then a gene analysis is performed on the sample data preprocessed. In the gene analysis, it is necessary to determine whether a required library file is in a gene shared memory of library files in gene analysis; if yes, the required library file is obtained from the gene shared memory, and mapped to the gene analysis corresponding to the sample data to complete the corresponding analysis. In the gene shared memory based gene analysis method the gene shared memory mechanism is used to establish indexes for gene analysis (for example comprises alignment analysis, variant calling analysis, annotation analysis and so on), and then stores files in a database (i.e. library files) required in the gene analysis in the gene shared memory. A library file can be conveniently mapped from the gene shared memory to a process of the gene analysis performed on the sample data. On one hand, the time and the I/O occupation for loading the library file from a hard disk are greatly reduced. On the other hand, the communications among multiple processes in the process of the gene analysis are facilitated and the repeatedly loading of the library file is avoid.

In some embodiment, the method further comprises: determining whether the required library file meets a load condition, in a case where the required library file in the gene analysis is not in the gene shared memory; and loading the required library file into the gene shared memory, in a case where the loading condition is met.

Specifically, if the required library file in the gene analysis is not in the gene shared memory, it is determined whether the required library file meets the load condition. The required library file can be loaded into the gene shared memory if the loading condition is met. On the one hand, it is faster and more efficient to load the required library file into the gene shared memory and then obtain the required library file from the gene shared memory; on the other hand, it can also facilitate other sample data processes to use the required library file, which avoids a repeated loading.

In some embodiments, determining whether the required library file meets a load condition, in a case where the required library file in the gene analysis is not in the gene shared memory, and loading the required library file into the gene shared memory, in a case where the loading condition is met comprises:

acquiring information of the required library file and information of the gene shared memory, wherein the information of the required library file comprises a space required by the required library file and the number of historical load requests, and the information of the gene shared memory comprises a remaining space of the gene shared memory; and if the number of historical load requests is greater than a first preset number, and the space required by the required library file is less than the remaining space of the gene shared memory, loading the required library file into the gene shared memory.

The information of the required library file refers to information related to the required library file, which can comprises a type of the required library file, a size of the required library file, a space required by the required library file, the number of historical load requests and a load request frequency of the required library file, etc. Information of the gene shared memory refers to information related to the gene shared memory, mainly comprising a size of the gene shared memory, a remaining space of the gene shared memory, etc.

A first preset number is a preset value, which can be used to reflect an importance of a library file to a certain extent. That is, if the number of historical load requests is greater than the first preset number, it indicates that the required library file is needed or used frequently, i.e., the required library file is important in the gene analysis, and can be loaded into the gene shared memory, so as to facilitate the use for other sample data. After determining the importance of the required library file, it is further necessary to determine whether the remaining space of gene shared memory is enough to store the required library file, that is, determine whether the space required by the required library file is less than the remaining space of gene shared memory. If so, the required library file can be directly loaded into gene shared memory.

In some embodiments, the information of the required library file further comprises a load request frequency of the required library file, the information of the gene shared memory further comprises load request frequencies of all library files; determining whether the required library file meets a load condition, and loading the required library file into the gene shared memory, in a case where the loading condition is met further comprises: if the number of historical load requests is greater than the first preset number, and the space required by the required library file is greater than the remaining space of the gene shared memory, ranking the required library file and the all library files in an order of priority according to the load request frequency of the required library file and the load request frequencies of the all library files to obtain a load request frequency priority of each library file; if the load request frequency priority of the required library file is higher than that of a library file in the gene shared memory, and if the remaining space of the gene shared memory after deleting the library file with a lower load request frequency priority in the gene shared memory is greater than or equal to the space required by the required library file, deleting the library file with the lower load request frequency priority in the gene shared memory; and loading the required library file into the gene shared memory.

Specifically, if it is determined that the space required for the required library file is greater than the remaining space of the gene shared memory, it indicates that the remaining space of the gene shared memory is not enough to store the required library file; in this case, it is necessary to compare the required library file with the library files already stored in the gene shared memory, delete a library file with a low load request frequency according to the load request frequency priorities of the library files, and then load the required library file into the gene shared memory.

In the embodiments, the required library file and the library files stored in the gene shared memory are ranked in an order of priority mainly according to the load request frequency of each library file. If the load request frequency priority of the required library file is higher than that of a library file in the gene shared memory, the library file in the gene shared memory is deleted to load the required library file into the gene shared memory. The sizes of all the library files are taken into comprehensive consideration in the above process. It is only necessary to ensure that the memory occupied by the deleted library file is sufficient to store the required library file.

In this way, when the required library file in the process of the gene analysis is not in the gene shared memory, the library file can be loaded into the gene shared memory first, so as to improve the efficiency of a subsequent calculation.

In some embodiments, the gene analysis comprises an alignment analysis, a variation analysis and an annotation analysis; the method further comprises: performing the alignment analysis, the variation analysis, and the annotation analysis on the sample data preprocessed in sequence, wherein in a case where the sample data preprocessed comprises multiple groups of sample data, the multiple groups of sample data are in a same step or different steps of the gene analysis at a time.

In the embodiments, the method of the gene analysis comprises the alignment analysis, the variation analysis and the annotation analysis. However, there is usually a sequence requirement in the process of the gene analysis, that is, the alignment analysis is generally carried out first, followed by the variation analysis, and then the annotation analysis. However, when there are multiple groups of sample data, each group of sample data can be in a same step or different steps of the gene analysis. For example, sample data 1 can be in an alignment analysis, sample data 2 can be in a variation analysis, and sample data 3 can be in an annotation analysis. It is also possible for sample data 1, sample data 2 and sample data 3 to be in an alignment analysis, a variation analysis or an annotation analysis at the same time. Multiple groups of sample data can be processed at the same time by using the method, which can further improve the data processing speed.

In some embodiments, the gene analysis further comprises a sorting analysis and a marking-duplicate analysis, wherein after performing the alignment analysis, the variation analysis, and the annotation analysis on the sample data preprocessed in sequence, the method further comprises : labeling the sample data after the alignment analysis with a position tag; and performing the sorting analysis and the marking-duplicate analysis by module on the sample data labeled.

Specifically, the gene analysis further comprises the sequencing analysis and the marking-duplicate analysis; labeling the sample data after the alignment analysis with a position tag is to add a position-related tag to a file after a comparison, so that the sequencing analysis and the marking-duplicate analysis can be performed by module, and more efficient multi-threaded sorting can be increased to the sequencing analysis and the marking-duplicate analysis.

In some embodiments, the method further comprises: connecting some or all steps of the gene analysis by a use of memory.

Specifically, several steps or all steps in processes of comparison, sorting, marking-duplicate and variant calling in the process of the gene analysis can be connected by the use of memory. Sam/bam files outputted intermediately can be reduced by connecting each step by the use of memory which reduces the I/O occupation.

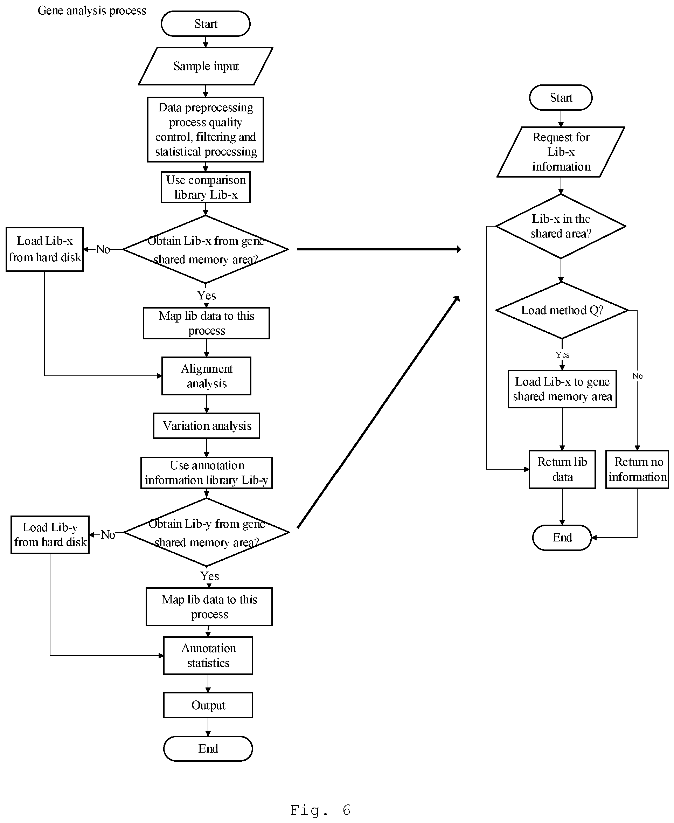

For ease of understanding, a detailed embodiment is given below. Fig. 6 shows the whole process of a gene analysis and a process in the gene shared memory area. The process of the gene analysis is as follows: after samples are input, the data of each sample is preprocessed, and then whether a library file required for an alignment analysis is loaded into the gene shared memory area is determined; if yes, the alignment analysis is started, or if not, the library file is loaded from a hard disk to perform the alignment analysis; the process of the alignment analysis is synthesized as a flexible step by a memory connection and an algorithm optimization; then the variant calling is performed, and whether a library file of annotation information has been loaded into the gene shared memory is determined; if yes, an annotation statistics is started, or if not, the library file is loaded from a hard disk for the annotation statistics; the analysis process is ended.

A process in the gene shared memory area is as follows: if there is a request for information of library lib-x (i.e. a required library file), whether the required library file is in the gene shared memory area is determined; if yes, library data is feedback, and the process is ended; if the required library file is not in the gene shared memory area, whether to load the required library file through a load method Q is determined; if yes, the required library file is loaded into the gene shared memory area, the library data is return, and the process is ended; if the required library file is not to be loaded through the load method Q, no information is returned and the process is ended.

The specific steps of the load method Q are as follows: 1. a type and a size of the required library file are determined; 2. a record file is obtained; 3. a total memory size of the node, a size of the shared memory area, the number of historical load requests of library and the total number of historical load requests of all types of libraries are read from the record file; 4. the memory size of the node is updated from a hard disk to prevent the memory size of the node from changing; 5. the number of historical load requests of this type of library is increased by 1 (f_type+1); 6. the total number of historical load requests of the all types of libraries is increased by 1 (f_total+1); 7. whether the remaining space is enough to load the library is determined; 8. request frequencies (f_type/f_total) of all types of libraries in the record file are ranked in descending order, and a ranked linked list is returned; 9. whether the required library file has been loaded is determined; if the required library file has been loaded, a library index is returned; if the required library file has not been loaded and the number of historical load requests of this type of library is more than 10, its priority and rank position in all unloaded libraries are determined; 10. if the priority of this type of library exceeds that of a loaded library, the system predicts whether a sum of the sizes of the loaded libraries ranked after this type of library in the type_list meets a condition W of a size of memory for loading this type of library; if yes, these loaded libraries are unloaded in reverse order until the condition W is met; if not, no process is performed; 11. if the load condition is met, the record of the size of the shared memory area is updated; 12. otherwise, a case that the library has not been loaded because there is no sufficient memory to load the library is marked, and update it to the record file.

The format of the record file is given below:
M: 63492649171200
Len: 13492649171200
f_total: 100

| Type | Size | Loaded | The number of historical load requests f type | type_flag |
|---|---|---|---|---|
| Libx | 10000000000000 | Yes | 75 | 0 |
| Liby | 3492649171200 | Yes | 12 | 0 |
| Libw | 40000000000000 | No | 10 | 1 |
| Libz | 5000000 | No | 3 | 0 |

type_flag indicates the reason for not being loaded, wherein "1" indicates that the load priority of this type of library was ranked first and it was not loaded because of insufficient memory, and the type_flag of a loaded library is 0.

In addition, the pseudo code of the load method Q is as follows.

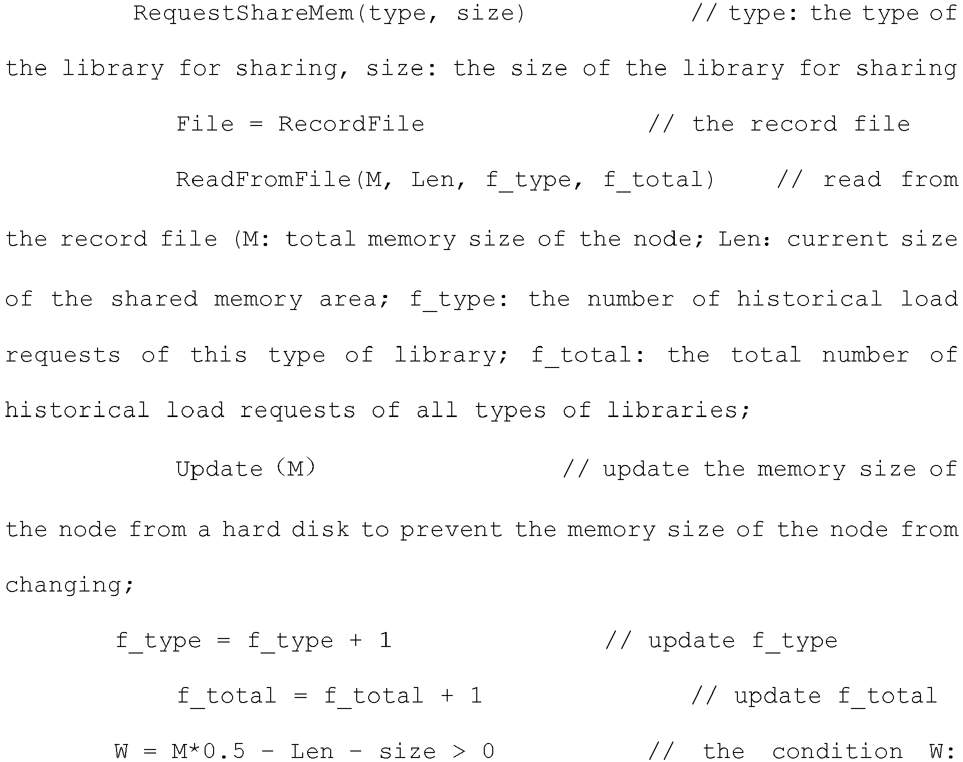

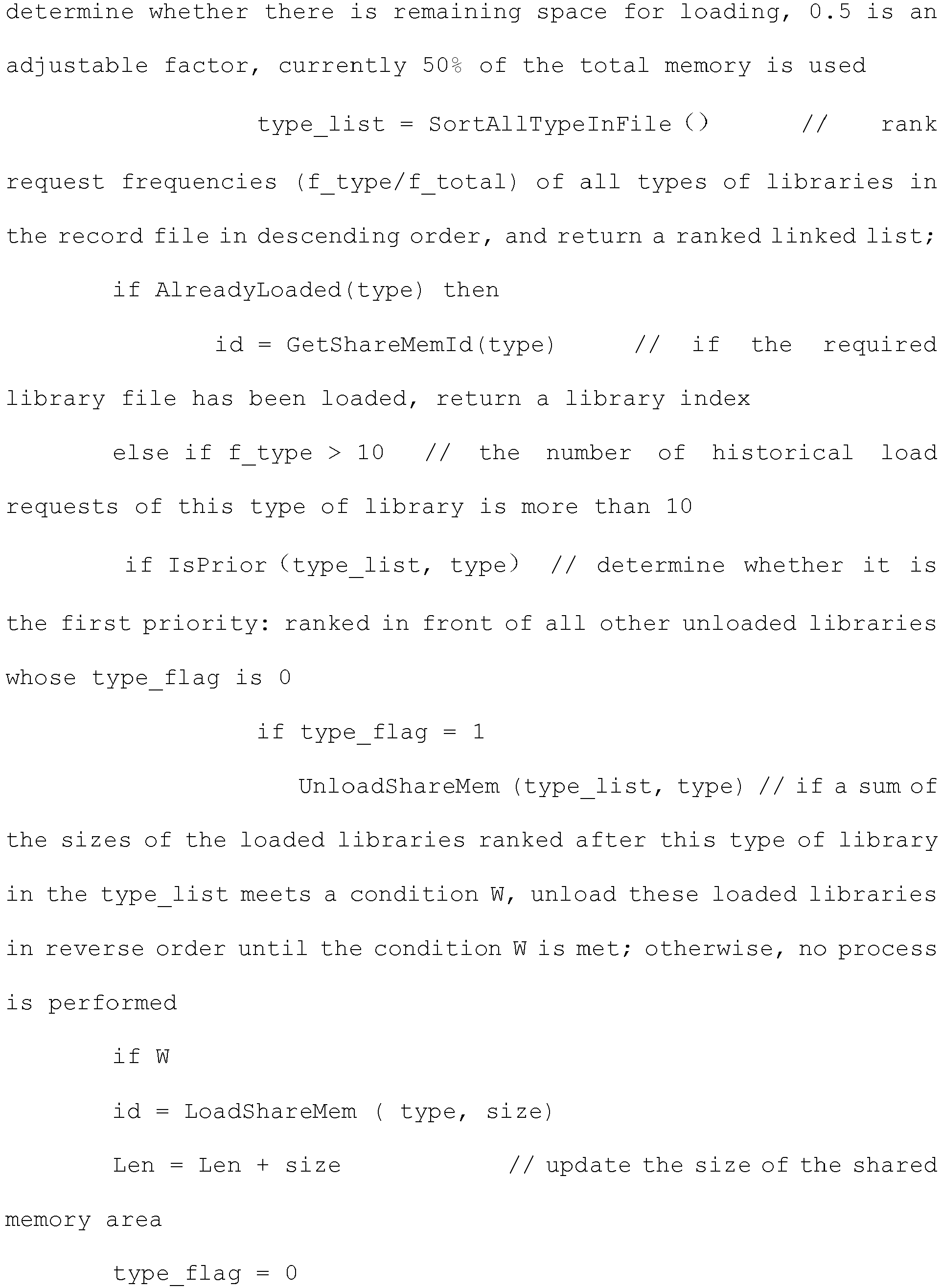

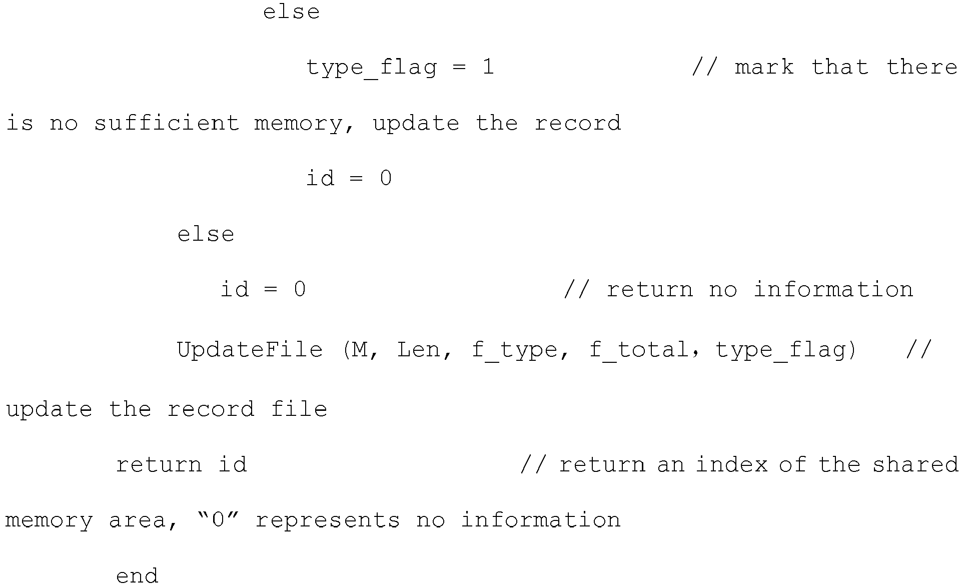

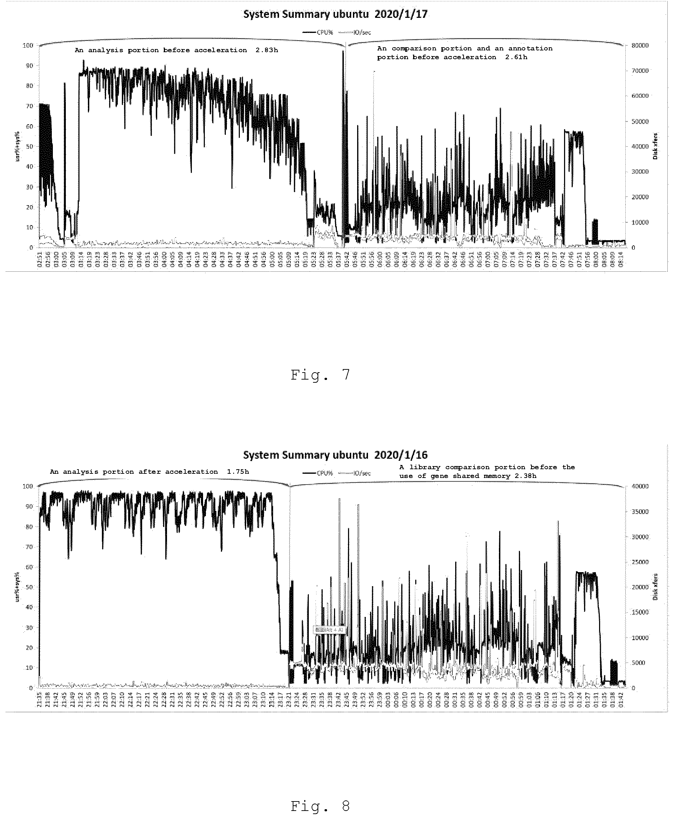

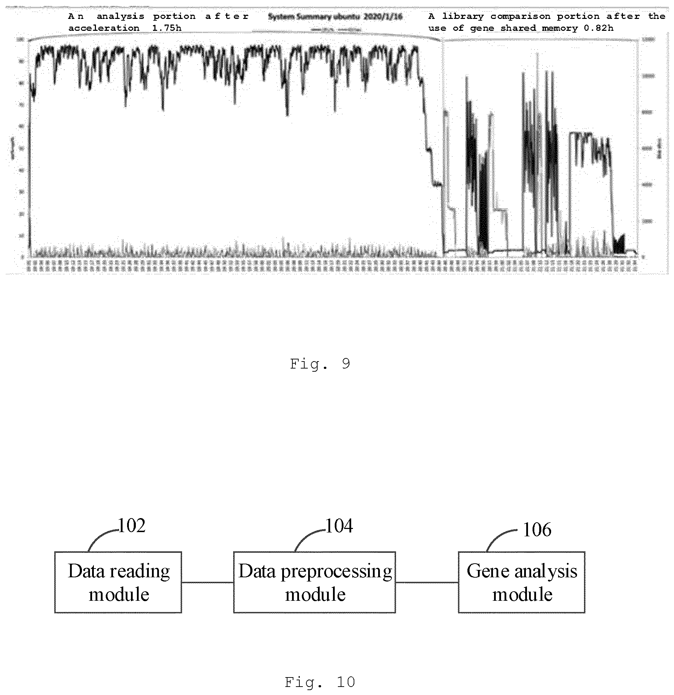

Some embodiments for showing effects:
In order to verify the effectiveness of the shared memory based gene analysis method in the embodiments of the disclosure, three gene analysis methods, namely method A (software without optimization (i.e. all steps of the gene analysis are not connected by a use of memory, and the steps are independent from each other) + without a use of the gene shared memory), method B (software with optimization (i.e. all steps of the gene analysis are connected by a use of memory) + without a use of the gene shared memory) and method C (software with optimization(i.e. all steps of the gene analysis are connected by a use of memory) + with a use of the gene shared memory) are given to compare CPU utilizations and I/O times of the methods. The results are shown in Figs. 7 to 9, wherein FIG. 7 shows an analysis result of the method A, Fig. 8 shows an analysis result of the method B, and Fig. 9 shows an analysis result of the C.

It can be seen from Figs. 7 to 9 that running time of an analysis portion of the method A before acceleration (i.e. each of a step of reading sample data and a step of preprocessing before the alignment analysis runs independently and the comparison is processed directly without the use of the gene shared memory) is 2.83 hours, and the CPU utilization fluctuates greatly. Running time of the comparison portion and an annotation portion before acceleration (i.e. the comparison and the annotation are processed directly without the use of the gene shared memory) is 2.61 hours, the CPU utilization is high, and the I/O sec (i.e. the number of transfers output to a physical disk per second) is high, indicating that the I/O utilization is high and the probability of blocking is high.

Running time of an analysis portion of the method B after acceleration (i.e. a step of reading sample data and a step of preprocessing before the alignment analysis are connected by the use of memory and the comparison is processed by using the gene shared memory) is 1.75 hours, and the CPU utilization fluctuates smaller than that of method A. Running time of a library comparison portion before the use of gene shared memory (i.e. the comparison is processed directly without the use of the gene shared memory) is 2.38 hours, the CPU utilization is high, and the I/O sec (i.e. the number of transfers output to a physical disk per second) is high, indicating that the I/O utilization is high and the probability of blocking is high.

Running time of an analysis portion of the method C after acceleration (i.e. a step of reading sample data and a step of preprocessing before the alignment analysis are connected by the use of memory and the comparison is processed by using the gene shared memory) is 1.75 hours, and the CPU utilization fluctuates smaller than that of method A (this portion is the same as method B) . Running time of a library comparison portion after the use of gene shared memory (i.e. the comparison is processed with the use of the gene shared memory) is 0.82 hours, the CPU utilization is high, and the I/O sec (i.e. the number of transfers output to a physical disk per second) is low, indicating that the I/O utilization is low and the probability of blocking is low.

Therefore, the method C is used for the gene analysis, that is, the gene analysis steps are connected by the use of memory. The method of adopting the gene shared memory in comparison, annotation and other processes can greatly reduce the time used for the gene analysis and reduce the I/O utilization rate, that is, reduce I/O blocking.

It should be understood that although the steps in the flowcharts of FIGS. 2, 4 and 6 are shown in order as indicated by the arrows, these steps are not necessarily performed in order as indicated by the arrows. Unless explicitly stated herein, the execution of these steps is not strictly limited in order, and these steps can be performed in other orders. Moreover, at least some steps in Figs. 2, 4, and 6 may comprise multiple sub-steps or multiple stages. These sub-steps or stages are not necessarily executed at the same time, but may be performed alternately with other steps or at least some sub-steps or stages of other steps.

In some embodiments, as shown in FIG. 10, there is provided a shared memory based gene analysis apparatus, comprising:

The data reading module 102 is configured to read sample data.

The data preprocessing module 104 is configured to preprocess the sample data.

The gene analysis module 106 is configured to perform a gene analysis on the sample data preprocessed, and determine whether a required library file in the gene analysis is in a gene shared memory; if yes, obtain the required library file from the gene shared memory, map the required library file to a process of the gene analysis of the sample data preprocessed, and complete a corresponding analysis.

In some embodiments, a library file loading module configured to determine whether the required library file meets a load condition, in a case where the required library file in the gene analysis is not in the gene shared memory; and load the required library file into the gene shared memory, in a case where the loading condition is met.

In some embodiments, the library file loading module comprises a library information and memory information acquisition module.

The library information and memory information acquisition module is configured to acquire information of the required library file and information of the gene shared memory, wherein the information of the required library file comprises a space required by the required library file and the number of historical load requests, and the information of the gene shared memory comprises a remaining space of the gene shared memory.

The library file loading module is configured to, if the number of historical load requests is greater than a first preset number, and the space required by the required library file is less than the remaining space of the gene shared memory, load the required library file into the gene shared memory.

In some embodiments, the information of the required library file further comprises a load request frequency of the required library file, the information of the gene shared memory further comprises load request frequencies of all library files; and the library file loading module further comprises a priority ranking module and a library file deleting module.

The priority sorting module is configured to, if the number of historical load requests is greater than the first preset number, and the space required by the required library file is greater than the remaining space of the gene shared memory, rank the required library file and the all library files in an order of priority according to the load request frequency of the required library file and the load request frequencies of the all library files to obtain a load request frequency priority of each library file.

The library file deleting module is configured to, if the load request frequency priority of the required library file is higher than that of a library file in the gene shared memory, and if the remaining space of the gene shared memory after deleting the library file with a lower load request frequency priority in the gene shared memory is greater than or equal to the space required by the required library file, delete the library file with the lower load request frequency priority in the gene shared memory.
the library file loading module is further configured to load the required library file into the gene shared memory.

In some embodiments, the apparatus further comprises: a gene shared memory setting module configured to set the gene shared memory for library files used in gene analysis, set a size of the gene shared memory, the number of library files that can be accommodated, a name of each library file and a size offset of the each library file.

The library file loading module is further configured to load library files commonly used in gene analysis into the gene shared memory according to the size of the gene shared memory, the number of library files that can be accommodated, the name of the each library file and the size offset of the each library file.

In some embodiments, the gene analysis comprises an alignment analysis, a variation analysis and an annotation analysis.

The gene analysis module is configured to perform the alignment analysis, the variation analysis, and the annotation analysis on the sample data preprocessed in sequence, wherein in a case where the sample data preprocessed comprises multiple groups of sample data, the multiple groups of sample data are in a same step or different steps of the gene analysis at a time.

In some embodiments, the gene analysis further comprises a sorting analysis and a marking-duplicate analysis, and the apparatus further comprises: a sorting and marking-duplicate module configured to label the sample data after the alignment analysis with a position tag; and perform the sorting analysis and the marking-duplicate analysis by module on the sample data labeled.

In some embodiments, the apparatus further comprises: a memory connection module configured to connect some or all steps of the gene analysis by a use of memory.

In some embodiments, the data preprocessing module is further a quality control, a filtering operation and a statistical process on the sample data perform a quality control, a filtering operation and a statistical process on the sample data.

For the specific definition of the shared memory based gene analysis apparatus, please refer to the definition of the shared memory based gene analysis method described above, which will not be repeated here. All or some of the modules in the shared memory based gene analysis apparatus can be realized by software, hardware, or a combination thereof. The above modules can be embedded in or independent of a processor of a computer device in the form of hardware, or stored in the memory in the computer device in the form of software, so as to facilitate the processor to call and execute the corresponding operations of the above modules.

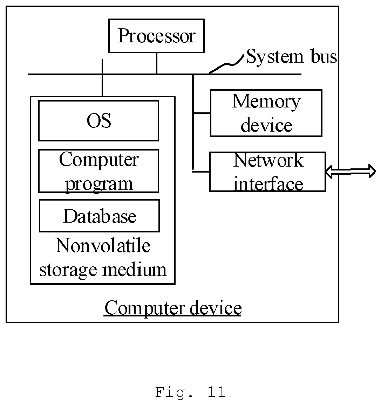

In some embodiments, a computer device is provided, which may be a server, and its internal structure may be as shown in FIG. 11. The computer device comprises a processor, a memory, a network interface and a database connected through a system bus . The processor of the computer device is used to provide computing and control capabilities. The memory of the computer device comprises a nonvolatile storage medium and a memory device. The nonvolatile storage medium stores an operating system, a computer program, and a database. The memory device provides an environment for the operation of the operating system and the computer program in nonvolatile storage medium. The database of the computer device is used to store the data of a resistance equivalent model and equivalent sub models, as well as the equivalent resistance, working resistance and contact resistance obtained during calculation. The network interface of the computer device is used to communicate with external terminals through network connection. The computer program is executed by the processor to implement a shared memory based gene analysis method.

Those skilled in the art can understand that the structure shown in FIG. 11 is only a block diagram of some structures related to the scheme of this application, and does not constitute a limitation on the computer device to which the scheme of this application is applied. The specific computer device may comprise more or fewer components than those shown in the Figure, or combine some components, or have different component arrangements.

In some embodiments, a computer device is provided, comprising a processor, a memory, and a computer program stored in the memory and executable by the processor, which when executing the computer program implements the following steps: reading sample data and preprocessing the sample data; performing a gene analysis on the sample data preprocessed, and determining whether a required library file in the gene analysis is in a gene shared memory; if yes, obtaining the required library file from the gene shared memory, mapping the required library file to a process of the gene analysis of the sample data preprocessed, and completing a corresponding analysis.

In some embodiments, the processor when executing the computer program further implements the following steps: determining whether the required library file meets a load condition, in a case where the required library file in the gene analysis is not in the gene shared memory; and loading the required library file into the gene shared memory, in a case where the loading condition is met.

In some embodiments, the processor when executing the computer program further implements a step of: determining whether the required library file meets a load condition, in a case where the required library file in the gene analysis is not in the gene shared memory, and loading the required library file into the gene shared memory, in a case where the loading condition is met comprises: acquiring information of the required library file and information of the gene shared memory, wherein the information of the required library file comprises a space required by the required library file and the number of historical load requests, and the information of the gene shared memory comprises a remaining space of the gene shared memory; and if the number of historical load requests is greater than a first preset number, and the space required by the required library file is less than the remaining space of the gene shared memory, loading the required library file into the gene shared memory.

In some embodiments, the processor when executing the computer program further implements the following step: the information of the required library file further comprises a load request frequency of the required library file, the information of the gene shared memory further comprises load request frequencies of all library files in the gene shared memory; determining whether the required library file meets a load condition, and loading the required library file into the gene shared memory, in a case where the loading condition is met further comprises: if the number of historical load requests is greater than the first preset number, and the space required by the required library file is greater than the remaining space of the gene shared memory, ranking the required library file and the all library files in an order of priority according to the load request frequency of the required library file and the load request frequencies of the all library files to obtain a load request frequency priority of each library file; if the load request frequency priority of the required library file is higher than that of a library file in the gene shared memory, and if the remaining space of the gene shared memory after deleting the library file with a lower load request frequency priority in the gene shared memory is greater than or equal to the space required by the required library file, deleting the library file with the lower load request frequency priority in the gene shared memory; and if the number of historical load requests is greater than a first preset number, and the space required by the required library file is less than the remaining space of the gene shared memory, loading the required library file into the gene shared memory.

In some embodiments, the processor when executing the computer program further implements the following step: the information of the required library file further comprises a load request frequency of the required library file, the information of the gene shared memory further comprises load request frequencies of all library files; determining whether the required library file meets a load condition, and loading the required library file into the gene shared memory, in a case where the loading condition is met further comprises: if the number of historical load requests is greater than the first preset number, and the space required by the required library file is greater than the remaining space of the gene shared memory, ranking the required library file and the all library files in an order of priority according to the load request frequency of the required library file and the load request frequencies of the all library files to obtain a load request frequency priority of each library file; if the load request frequency priority of the required library file is higher than that of a library file in the gene shared memory, and if the remaining space of the gene shared memory after deleting the library file with a lower load request frequency priority in the gene shared memory is greater than or equal to the space required by the required library file, deleting the library file with the lower load request frequency priority in the gene shared memory; and loading the required library file into the gene shared memory.

In some embodiments, the processor when executing the computer program further implements the following steps: setting the gene shared memory for library files used in gene analysis, setting a size of the gene shared memory, the number of library files that can be accommodated, a name of each library file and a size offset of the each library file; and loading library files commonly used in gene analysis into the gene shared memory according to the size of the gene shared memory, the number of library files that can be accommodated, the name of the each library file and the size offset of the each library file.

In some embodiments, the processor when executing the computer program further implements the following step: the gene analysis comprises an alignment analysis, a variation analysis and an annotation analysis, and the processor when executing the computer program further implements the following step: performing the alignment analysis, the variation analysis, and the annotation analysis on the sample data preprocessed in sequence, wherein in a case where the sample data preprocessed comprises multiple groups of sample data, the multiple groups of sample data are in a same step or different steps of the gene analysis at a time.

In some embodiments, the processor when executing the computer program further implements the following step: the gene analysis further comprises a sorting analysis and a marking-duplicate analysis, wherein after performing the alignment analysis, the variation analysis, and the annotation analysis on the sample data preprocessed in sequence, the processor when executing the computer program further implements the following steps: labeling the sample data after the alignment analysis with a position tag; and performing the sorting analysis and the marking-duplicate analysis by module on the sample data labeled.

In some embodiments, the processor when executing the computer program further implements the following step: connecting some or all steps of the gene analysis by a use of memory.

In some embodiments, the processor when executing the computer program further implements the following step: preprocessing the sample data comprises: performing a quality control, a filtering operation and a statistical process on the sample data.

Some embodiments provide a computer-readable storage medium on which a computer program is stored, which when executed by a processor implements the following steps: reading sample data and preprocessing the sample data; performing a gene analysis on the sample data preprocessed, and determining whether a required library file in the gene analysis is in a gene shared memory; if yes, obtaining the required library file from the gene shared memory, mapping the required library file to a process of the gene analysis of the sample data preprocessed, and completing a corresponding analysis.

In some embodiments, the processor when executing the computer program further implements the following steps: determining whether the required library file meets a load condition, in a case where the required library file in the gene analysis is not in the gene shared memory; and loading the required library file into the gene shared memory, in a case where the loading condition is met.

In some embodiments, the computer program when executed by a processor implements the following steps: determining whether the required library file meets a load condition, in a case where the required library file in the gene analysis is not in the gene shared memory, and loading the required library file into the gene shared memory, in a case where the loading condition is met comprises: acquiring information of the required library file and information of the gene shared memory, wherein the information of the required library file comprises a space required by the required library file and the number of historical load requests, and the information of the gene shared memory comprises a remaining space of the gene shared memory; and if the number of historical load requests is greater than a first preset number, and the space required by the required library file is less than the remaining space of the gene shared memory, loading the required library file into the gene shared memory.

In some embodiments, the computer program when executed by a processor implements the following steps: the information of the required library file further comprises a load request frequency of the required library file, the information of the gene shared memory further comprises load request frequencies of all library files in the gene shared memory; determining whether the required library file meets a load condition, and loading the required library file into the gene shared memory, in a case where the loading condition is met further comprises: if the number of historical load requests of the required library file is greater than the first preset number, and the space required by the required library file is greater than the remaining space of the gene shared memory, ranking the required library file and the all library files in an order of priority according to the load request frequency of the required library file and the load request frequencies of the all library files to obtain a load request frequency priority of each library file; if the load request frequency priority of the required library file is higher than that of a library file in the gene shared memory, and if the remaining space of the gene shared memory after deleting the library file with a lower load request frequency priority in the gene shared memory is greater than or equal to the space required by the required library file, deleting the library file with the lower load request frequency priority in the gene shared memory; and if the number of historical load requests of the required library file is greater than a first preset number, and the space required by the required library file is less than the remaining space of the gene shared memory, loading the required library file into the gene shared memory.

In some embodiments, the computer program when executed by a processor implements the following steps: the information of the required library file further comprises a load request frequency of the required library file, the information of the gene shared memory further comprises load request frequencies of all library files; determining whether the required library file meets a load condition, and loading the required library file into the gene shared memory, in a case where the loading condition is met further comprises: if the number of historical load requests is greater than the first preset number, and the space required by the required library file is greater than the remaining space of the gene shared memory, ranking the required library file and the all library files in an order of priority according to the load request frequency of the required library file and the load request frequencies of the all library files to obtain a load request frequency priority of each library file; if the load request frequency priority of the required library file is higher than that of a library file in the gene shared memory, and if the remaining space of the gene shared memory after deleting the library file with a lower load request frequency priority in the gene shared memory is greater than or equal to the space required by the required library file, deleting the library file with the lower load request frequency priority in the gene shared memory; and loading the required library file into the gene shared memory.

In some embodiments, the computer program when executed by a processor further implements the following steps: setting the gene shared memory for library files used in gene analysis, setting a size of the gene shared memory, the number of library files that can be accommodated, a name of each library file and a size offset of the each library file; and loading library files commonly used in gene analysis into the gene shared memory according to the size of the gene shared memory, the number of library files that can be accommodated, the name of the each library file and the size offset of the each library file.

In some embodiments, the computer program when executed by a processor further implements the following steps: the gene analysis comprises an alignment analysis, a variation analysis and an annotation analysis, and the computer program when executed by a processor further implements the following step: performing the alignment analysis, the variation analysis, and the annotation analysis on the sample data preprocessed in sequence, wherein in a case where the sample data preprocessed comprises multiple groups of sample data, the multiple groups of sample data are in a same step or different steps of the gene analysis at a time.

In some embodiments, the computer program when executed by a processor further implements the following steps: the gene analysis further comprises a sorting analysis and a marking-duplicate analysis, wherein after performing the alignment analysis, the variation analysis, and the annotation analysis on the sample data preprocessed in sequence, the computer program when executed by a processor further implements the following steps: labeling the sample data after the alignment analysis with a position tag; and performing the sorting analysis and the marking-duplicate analysis by module on the sample data labeled.

In some embodiments, the computer program when executed by a processor further implements the following step: connecting some or all steps of the gene analysis by a use of memory.

In some embodiments, the computer program when executed by a processor further implements the following step: preprocessing the sample data comprises: performing a quality control, a filtering operation and a statistical process on the sample data.

As understood by those skilled in the art, all or part of the steps for carrying out the method in the above embodiments can be completed by hardware or a program instructing the related hardware, wherein the program can be stored in a computer readable nonvolatile storage medium; the program when executed can carry out the steps of the embodiments of the above methods; Any reference to memory, storage, database or other media used in the embodiments provided by the present application may comprise nonvolatile and/or volatile memory. The nonvolatile memory may comprise read only memory (ROM), programmable ROM (PROM), electrically programmable ROM (EPROM), electrically erasable programmable ROM (EEPROM), or flash memory. The volatile memory may comprise random access memory (RAM) or external cache memory. As an illustration rather than a limitation, RAM is available in various forms, such as static RAM (SRAM), dynamic RAM (DRAM), synchronous DRAM (SDRAM), dual data rate SDRAM(DDRSDRAM), enhanced SDRAM (ESDRAM), synchronous link DRAM (SLDRAM), Rambus direct RAM (RDRAM), direct memory bus dynamic RAM (DRDRAM) and Rambus dynamic RAM (RDRAM), etc.

The technical features of the above embodiments can be combined arbitrarily. In order to make the description concise, all possible combinations of the various technical features in the embodiments are not described, but should be regarded as within the scope of this description, as long as there is no contradiction in the combinations of these technical features.

The aforesaid embodiments merely present several embodiments of the present application. However, the relatively specific and detailed descriptions thereof cannot therefore be construed as limiting the scope of the present application. It shall be pointed out that a person skilled in the art is capable of making various modifications and improvements without departing from the concept of the present application. Such modifications and improvements shall be regarded as within the protection scope of the present application. Therefore, the protection scope of the present application shall be determined by the terms of the claims.

## Claims

1. A shared memory based gene analysis method, **characterized by**, comprising:
reading sample data and preprocessing the sample data;
performing a gene analysis on the sample data preprocessed, and determining whether a required library file in the gene analysis is in a gene shared memory;
if yes, obtaining the required library file from the gene shared memory, mapping the required library file to a process of the gene analysis of the sample data preprocessed, and completing a corresponding analysis.

2. The shared memory based gene analysis method according to claim 1, **characterized by**, further comprising:
determining whether the required library file meets a load condition, in a case where the required library file in the gene analysis is not in the gene shared memory; and
loading the required library file into the gene shared memory, in a case where the loading condition is met.

3. The shared memory based gene analysis method according to claim 2, **characterized in that** determining whether the required library file meets a load condition, in a case where the required library file in the gene analysis is not in the gene shared memory, and loading the required library file into the gene shared memory, in a case where the loading condition is met comprises:
acquiring information of the required library file and information of the gene shared memory, wherein the information of the required library file comprises a space required by the required library file and the number of historical load requests, and the information of the gene shared memory comprises a remaining space of the gene shared memory; and
if the number of historical load requests is greater than a first preset number, and the space required by the required library file is less than the remaining space of the gene shared memory, loading the required library file into the gene shared memory.

4. The shared memory based gene analysis method according to claim 3, **characterized in that** the information of the required library file further comprises a load request frequency of the required library file, the information of the gene shared memory further comprises load request frequencies of all library files; determining whether the required library file meets a load condition, and loading the required library file into the gene shared memory, in a case where the loading condition is met further comprises:
if the number of historical load requests is greater than the first preset number, and the space required by the required library file is greater than the remaining space of the gene shared memory, ranking the required library file and the all library files in an order of priority according to the load request frequency of the required library file and the load request frequencies of the all library files to obtain a load request frequency priority of each library file;
if the load request frequency priority of the required library file is higher than that of a library file in the gene shared memory, and if the remaining space of the gene shared memory after deleting the library file with a lower load request frequency priority in the gene shared memory is greater than or equal to the space required by the required library file, deleting the library file with the lower load request frequency priority in the gene shared memory; and
loading the required library file into the gene shared memory.

5. The shared memory based gene analysis method according to any one of claims 1 to 4, **characterized by**, further comprising:
setting the gene shared memory for library files used in gene analysis, setting a size of the gene shared memory, the number of library files that can be accommodated, a name of each library file and a size offset of the each library file; and
loading library files commonly used in gene analysis into the gene shared memory according to the size of the gene shared memory, the number of library files that can be accommodated, the name of the each library file and the size offset of the each library file.

6. The shared memory based gene analysis method according to claim 1, **characterized in that** the gene analysis comprises an alignment analysis, a variation analysis and an annotation analysis, and the method further comprises:
performing the alignment analysis, the variation analysis, and the annotation analysis on the sample data preprocessed in sequence, wherein in a case where the sample data preprocessed comprises multiple groups of sample data, the multiple groups of sample data are in a same step or different steps of the gene analysis at a time.

7. The shared memory based gene analysis method according to claim 6, **characterized in that** the gene analysis further comprises a sorting analysis and a marking-duplicate analysis, wherein after performing the alignment analysis, the variation analysis, and the annotation analysis on the sample data preprocessed in sequence, the method further comprises:
labeling the sample data after the alignment analysis with a position tag; and performing the sorting analysis and the marking-duplicate analysis by module on the sample data labeled.

8. The shared memory based gene analysis method according to claim 7, **characterized by**, further comprising:
connecting some or all steps of the gene analysis by a use of memory.

9. The shared memory based gene analysis method according to any one of claims 6 to 8, **characterized in that** preprocessing the sample data comprises:
performing a quality control, a filtering operation and a statistical process on the sample data.

10. A shared memory based gene analysis apparatus, **characterized by**, comprising:
a data reading module configured to read sample data;
a data preprocessing module configured to preprocess the sample data; and
a gene analysis module configured to perform a gene analysis on the sample data preprocessed, and determine whether a required library file in the gene analysis is in a gene shared memory; if yes, obtain the required library file from the gene shared memory, map the required library file to a process of the gene analysis of the sample data preprocessed, and complete a corresponding analysis.

11. A computer device comprising: a memory, a processor, and a computer program stored on the memory and executable on the processor, **characterized in that** the processor when executing the computer program implements the steps of the method according to any one of claims 1 to 9.

12. A computer-readable storage medium on which a computer program is stored, **characterized in that** the computer program when executed by a processor implements the steps of the method according to any one of claims 1 to 9.
